(19) Europäisches Patentamt
European Patent Office
Office européen des brevets

(11) Publication number: **0 365 134 A1**

(12) **EUROPEAN PATENT APPLICATION**

(21) Application number: 89309201.5

(22) Date of filing: 11.09.89

(51) Int. Cl.5: A61K 37/64 , A61K 31/55 , //(A61K31/55,31:40)

Claims for the following Contracting States: ES + GR.

(30) Priority: 16.09.88 US 245889

(43) Date of publication of application: 25.04.90 Bulletin 90/17

(84) Designated Contracting States: AT BE CH DE ES FR GB GR IT LI LU NL SE

(71) Applicant: SMITHKLINE BEECHAM CORPORATION
One Franklin Plaza
Philadelphia Pennsylvania 19103(US)

(72) Inventor: Brennan, Francis Thomas
907 Sara Drive
Springfield Pennsylvania 19064(US)
Inventor: Kinter, Lewis Boardman
200 East Eagle Road
Havertown Pennsylvania 19083(US)
Inventor: Kavanagh, Bernard Joseph
2544 Pettit Road
Pennsauken New Jersey 08109(US)
Inventor: Wiebelhaus, Virgil, Daniel

Deceased(US)

(74) Representative: Giddings, Peter John, Dr. et al
Smith Kline & French Laboratories Ltd.
Corporate Patents Mundells
Welwyn Garden City Hertfordshire AL7 1EY(GB)

(54) **Synergistic compositions of renal dopaminergic agent and angiotensin converting enzyme inhibitors.**

(57) Compositions useful for improving kidney function and treating hypertension are comprised of a 6-halo-7,8-dihydroxy-1-(4-hydroxyphenyl)-2,3,4,5-tetrahydro-1H-3-benzazepine combined with angiotensin converting enzyme inhibitors, advantageously captopril. Preferably the compositions are administered orally.



Xerox Copy Centre

# SYNERGISTIC COMPOSITIONS OF RENAL DOPAMINERGIC AGENT AND ANGIOTENSIN CONVERTING ENZYME INHIBITORS

This invention comprises a composition containing certain selected benzazepine compounds which have specific renal vasodilator activity but which do not elevate glomerular filtration rate (GFR), i.e., a dopamine-like agent combined with one or more known compounds which are angiotensin converting enzyme (ACE), inhibitors. The composition which contains this new combination of medicinal agents demonstrates an improved efficiency of kidney function and an improved antihypertensive effect. An unexpected increase in the glomerular filtration rate together with an increase in renal plasma flow (RPF) has been demonstrated with the combination product of this invention.

## Background

GFR and RPF are two very important parameters which maintain or improve renal function. Improved kidney function is beneficial in hypertensive patients and patients with diminished renal function. Glomerular filtration is the principle step in the regulation of the excretion of water and circulating solutes in mammals. Increases in GRF lead to increases in water and solute excretion, decreases in GFR are associated with water and solute retention. Reduced GFR is associated with both acute and chronic disease processes and in surgical and traumatic injury. Loss of GFR may result in oliguria or anuria and fluid and solute retention leading to uremia and edema. These would require extracorporal dialysis if GFR is not restored. A drug or drug combination which could increase GFR while effecting renal and systemic vasodilation would present specific advantages in congestive heart failure associated with uremia and acute renal failure associated with trauma, surgery or shock. The drug or combination could also have therapeutic utility in chronic renal failure, an area currently devoid of therapeutic agents.

The components of the composition of this invention have been described in the literature as having antihypertensive activity. The renal vasodilator ingredients in the claimed compositions are described in U.S. Patent No. 4,197,297. The ACE inhibitors are also well known to the art as systemic vasodilators by virtue of their effect to block synthesis of the potent endogenous vasconstrictor hormone, angiotensin II. However, there is no prior art mention of the claimed compositions and method for elevating GFR and improving kidney function known to the applicants.

## Description of the Invention

The renal vasodilator component of this combination will be a compound of the structure:

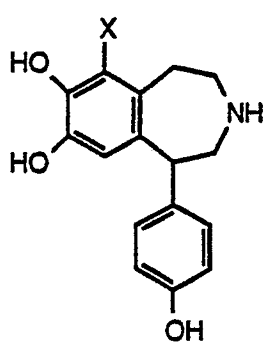

(I)

in which X is halo.

The nontoxic pharmaceutically acceptable acid addition salts of the compounds of Formula I are similarly useful in the compositions and methods of this invention. Examples of such salts are the hydrochloride, hydrobromide, sulfate, phosphate, sulfonate, methyl sulfonate, maleate or fumarate salts. Preferably the hydrochloride, hydrobromide or methyl sulfonate salts are employed. These salt forms are prepared by methods well known to the art.

It will be obvious to one skilled in the art that the compounds of Formula I may be present as diasteroisomers which may be resolved into d, 1 optical isomer. The separated stereoisomers are also useful and intended to be covered in this invention.

The 3-benzazepine compounds of Formula I are described in the prior literature to have an antihypertensive effect in subjects having, or prone to have, elevated blood pressure. The compounds are presumed to work by means of a specific drug action at the peripheral dopaminergic receptors, especially in the kidney, thereby increasing renal blood flow and decreasing renal vascular resistance. The quantity of the dopaminergic agent in the compositions of this invention will vary with the potency of its biological activity as well as with its pharmacodynamic characteristics. Such quantity is chosen from the dose ranges of the dopaminergic agent in the literature useful for treating patients. Generally, the quantity of dopaminergic agent in a dosage unit of this invention will be selected from the range of about 15-500 mg. Using, for example, 6-chloro-7,8-dihydroxy-1-(4-hydroxyphenyl)-2,3,4,5-tetrahydro-1H-3-benzazepine, as the methyl sulfonate salt, from about 25-300 mg of base equivalent is used in the composition. Preferably, the quantity of dopaminergic agent will be chosen from the range of 50-150 mg. The resulting composition is administered from 1-6 times a day. Other dopaminergic agents may be used, basing their unit dosages on this daily regimen with consideration of their quantitative efficacy compared with the compound.

The ACE inhibitor component of the compositions for improving kidney function of this invention will be any such standard agent known to the art such as, for example, captopril, enalapril, lisinopril and quinopril. The ACE inhibitors will be present in the composition in quantities within its normal dosage unit and daily regimen ranges as described in the medical literature. Captopril or one of its pharmaceutically acceptable acid addition salts is the preferred ACE inhibitor of this novel combination. Normally the ACE inhibitor will be selected from the range of about 1 mg to about 100 mg depending on its known potency. A particularly effective nontoxic quantity of the ACE inhibitor compound in a dosage unit will be from about 5 mg to about 50 mg, preferably 10 mg to about 25 mg.

As stated above, the active renal vasodilator and ACE inhibitor ingredients of the novel compositions are not used in any critical ratio but each is used at one of its clinically effective doses. The resulting compositions exhibit the full spectrum of the biological activity of each of the ingredients with the unexpected synergistic GFR effects discussed above and demonstrated hereafter.

It has been discovered that the administration to a subject in need of improvement in kidney function of a specifically acting renal vasodilating agent when combined with a ACE inhibitor agent, each administered in accepted clinically effective quantities, unexpectedly and significantly increases the glomerular filtration rate of the designated subject beyond that of simply adding the effects of the components. The unexpected biological effect has not been previously reported in the combination studies mentioned above. In addition to GFR synergism, the combination also increases renal plasma flow. The compositions of this invention are, therefore, useful in improving kidney function of the patient without increasing blood pressure or, even more so, in treating the hypertensive patient.

A modification of the renal function protocol, described in the prior art, was used for renal clearance studies in adult, trained, unanesthetized, fasted, female mongrel dogs, lightly restrained on their backs on a cradle-like board constructed specifically for this purpose. The effect of compound upon kidney function was determined by measurement of effective renal plasma flow (RPF) and glomerular filtration rate.

All dogs were given an oral water load of 500 ml of tap water thirty minutes prior to initiation of the study. Using a constant infusion pump, the dogs were infused intravenously at 3 ml/minute throughout the course of the experiment with a 4% mannitol-phosphate buffer solution, pH 7.4. Glomerular filtration rate was determined as the clearance of creatinine and effective renal plasma flow as the clearance of p-aminohippuric acid (PAH) These clearances were determined simultaneously by including 0.4% creatinine and 0.08% p-aminohippuric acid in the infusion. Suitable plasma levels of creatinine and p-aminohippuric acid were obtained by a thirty minute infusion of this solution prior to starting of the initial urine collection ($U_1$) In addition, 1.5 ml/kg of a 1% creatinine solution in phosphate buffer was injected intravenously fifteen minutes prior to collection of $U_1$. Replicate urine collections were obtained at ten minute intervals throughout the experiment. The bladder was emptied by an indwelling catheter and complete urine collections were assured by rinsing the bladder with 10 mls of warm water at the end of each collection interval. This procedure was followed by an air washout. Venous blood samples were drawn from an indwelling catheter at the mid-point of each clearance period.

Urine volumes were recorded at the time of collection. Plasma and urine samples were stored frozen, after collection, until analyzed. Analyses for creatinine and p-aminohippuric acid were made, by colorimetry, using standard AutoAnalyzer methodologies. These analyses were made simultaneously on each sample. Peak intensities were identified electronically, recorded on "on-line" teletype, stored and subsequently analyzed mathematically by time-shared computer against appropriate standards. Data were computed from

linear least square regression lines, and quality control standards were analyzed simultaneously to evaluate system reproducibility and reliability.

The bladder was emptied upon beginning the study and three control clearances ($U_1$-$U_3$, Phase 1) were obtained.

6-Chloro-7,8-dihydroxy-1-(4-hydroxyphenyl)-2,3,4,5-tetrahydro-1H-3-benzazepine as the methyl sulfonate salt was given orally by stomach tube at doses of 2.5, 5, 10 and 20 mg/kg of the base after Phase 1.

Captopril was given by the same route after the 6th clearance ($C_6$) at a dose of 5.0 mg/kg.

Post-drug clearance $C_4$-$C_6$, $C_7$-$C_9$, $C_{10}$-$C_{12}$ (Phases II, III and IV) were collected immediately, with no delay between $C_3$ and $C_4$. The total post-drug period observed was one and one-half hours. Four dogs were used for each treatment.

All compounds were weighed as free base content.

Following are results of testing the combinations using this protocol:

TABLE 1

Effects of Fenoldopam,** Captopril, and Combinations on Effective Renal Plasma Flow (ERPF) and Glomerular Filtration Rate (GFR) in Dogs

| | (n) | ERPF | GFR |
|---|---|---|---|
| | | ( % from control) | |
| Captopril | | | |
| 5 mg/kg | (3) | 4 ± 3 | -3 ± 2 |
| Fenoldopam | | | |
| 2.5 mg/kg | (4) | 34 ± 5 | -2 ± 2 |
| 5.0 | (8) | 47 ± 16 | 6 ± 5 |
| 10 | (4) | 30 ± 2 | -1 ± 2 |
| 20 | (4) | 44 ± 15 | 1 ± 3 |
| Fenoldopam Plus Captopril 5 mg/kg, p.o.) | | | |
| 2.5 mg/kg | (4) | 32 ± 6 | 15 ± 2* |
| 5 | (4) | 56 ± 5 | 11 ± 5 |
| 10 | (4) | 71 ± 15* | 14 ± 10 |
| 20 | (4) | 93 ± 7* | 20 ± 6* |

* Statistically significant compared with Fenoldopam alone.

** 6-Chloro-7,8-dihydroxy-1-(4-hydroxyphenyl)-2,3,4,5-tetrahydro-1H-3-benzazepine methyl sulfonate.

The data in the above table demonstrates the significant synergistic renal effects of the combination of this invention on the renal plasma flows as well as in particular, the glomerular filtration rates.

The dosage unit compositions of this invention are prepared by standard pharmaceutical techniques by incorporation into a pharmaceutical carrier in the form of a dosage unit such as a tablet, troche, capsule or powder for either suspension or solution. For example, an effective quality of a dopaminergic compound of Formula 1 selected from the dose range presented above together with an effective quantity of an ACE inhibitor agent are screened, sized and mixed together with a filler if need be, for example, talc, lactose, terra alba, magnesium stearate, agar, pectin, acacia, gelatin or stearic acid. The active ingredients and filler are mixed and filled into a hard gelatin capsule. Alternatively, the ingredients are tableted using lubricants and granulating agents. Also, liquid carriers can be used, for example, peanut oil, sesame oil, olive oil or water. Such mixtures are encapsulated into soft gelatin capsules or, in case of a sterile, isotonic solution for parenteral use, in a unit of multidose vial.

If the combination is desired to be in a time release formulation, the active ingredients may be coated

with glyceryl distearate, wax, polyvinylpyrrolidone, zein, ethylcellulose, castor wax, polymethacrylates, cellulose acetate butyrate, a cross-linked polymeric film, for example, one formed from prepolymers of an unsaturated dicarboxylic acid and an ethylene compound, or acid copolymers formed from acrylic or methacrylic acid monomers.

The dosage unit compositions are administered orally to patients who have subnormal kidney function, abnormally high blood pressure or who are prone to have high blood pressure, usually, form 1-6 times daily, preferably from 2-4 times daily. In certain cardiovascular emergencies, the combined compounds in pharmaceutically acceptable salt form may be dissolved or suspended in saline and administered parenterally. It will be appreciated that the antihypertensive compositions of this invention combine clinically acceptable doses of dopaminergic agent and an ACE inhibitor with consideration for the half-life and peak therapeutic effect of each. It may be desirable in certain patients for the primary care physician to administer the two agents individually to achieve the novel therapeutic effect described herein. Also, under certain circumstances, a third agent from the same or another class of compounds may be added such as an oral dosage unit containing clinically effective quantities of 6-chloro-1-(4-hydroxyphenyl)-7,8-dihydroxy-3,4,5-tetrahydro-1H-3-benzazepine methyl hydrochlorothiazide or triameterene.

The following examples are designed to illustrate the preparation and use of the compositions of this invention and are not to be construed as a limitation thereof.

| EXAMPLE 1 | |
|---|---|
| Ingredients | Amounts, mg |
| 6-chloro-7,8-dihydroxy-1-(4-hydroxyphenyl)-2,3,4,5-tetrahydro-1H-3-benzazepine methyl-sulfonate | 100 |
| Captopril | 10 |
| Sucrose | 25 |
| Calcium sulfate, dihydrate | 50 |
| Talc | 5 |
| Stearic acid | 3 |
| Starch | 10 |

The active ingredients, sucrose, and calcium sulfate are mixed, granulated using hot 10% gelatin solution, meshed and dried. After mixing with the remaining ingredients, the mixture is compressed into a scored tablet.

The scored tablet, per se or broken, is administered orally to a patient in need of improved kidney function from 2-4 times daily.

| EXAMPLE 2 | |
|---|---|
| Ingredients | Amounts, mg |
| 6-chloro-7,8-dihydroxy-1-(4-hydroxyphenyl)-2,3,4,5-tetrahydro-1H-3-benzazepine methyl-sulfonate as base | 50 |
| Captopril | 10 |
| Magnesium Stearate | 2 |
| Lactose | 200 |

The above ingredients are thoroughly mixed and placed into hard gelatin capsules. The capsules are administered orally to subjects in need of improved kidney function from 2 to 4 times daily.

**Claims**

1. A pharmaceutical composition comprising:
(a) a compound of the following formula (I)

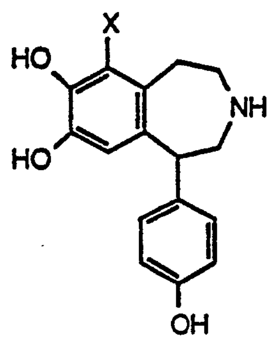

(I)

in which X is halo or a pharmaceutically acceptable acid addition salt; and
(b) a nontoxic clinically active quantity of an angiotensin converting enzyme inhibitor.

2. A composition of claim 1 in which the dopaminergic compound is 6-chloro-7,8-dihydroxy-1-(4-hydroxyphenyl)-2,3,4,5-tetrahydro-1H-3-benzazepine or a pharmaceutically acceptable acid addition salt thereof and the angiotensin converting enzyme inhibitor is captopril.

3. A composition of claim 2 in which the dopaminergic compound is present as its methyl sulfonate salt.

4. A composition of claim 2 in which the quantity of the dopaminergic compound is selected from about 50 to 150 mg and the quantity of the enzyme inhibitor is selected from about 10 to 25 mg.

5. A composition as claimed in any one of claims 1 to 4 in a form suitable for oral administration.

6. The use of a composition as claimed in any one of claims 1 to 5 in therapy.

7. The use of a composition as claimed in any one of claims 1 to 5 in improving kidney function.

8. The use of a composition as claimed in any one of claims 1 to 5 in the manufacture of a medicament for use in improving kidney function.

Claims for the following Contracting states ES, GR

1. A process for preparing a pharmaceutical composition which comprises bringing into association a compound of the following formula (I),

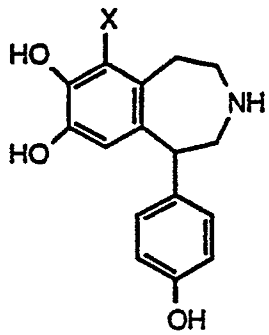

(I)

in which X is halo or a pharmaceutically acceptable acid addition salt, and a nontoxic clinically active quantity of an angiotensin converting enzyme inhibitor, together with a pharmaceutically acceptable carrier.

2. A process according to claim 1 in which the compound of formula (I) is 6-chloro-7,8-dihydroxy-1-(4-hydroxyphenyl)-2,3,4,5-tetrahydro-1H-3-benzazepine or a pharmaceutically acceptable acid addition salt thereof and the angiotensin converting enzyme inhibitor is captopril.

3. A process according to claim 2 in which the compound of formula (I) is present as its methyl sulfonate salt.

4. A process according to claim 2 in which the quantity of compound of formula (I) is selected from about 50 to 150 mg and the quantity of the angiotensin converting enzyme inhibitor is selected from about 10 to 25 mg.

5. A process as claimed in any one of claims 1 to 4 in which the composition prepared is in a form suitable for oral administration.

European Patent Office

# EUROPEAN SEARCH REPORT

Application Number

EP 89 30 9201

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PROC. WEST. PHARMACOL. SOC. vol. 31, 1988, p. 87-90, Los Angeles, Calif. USA: D.L. McCLELLAND & R.P. ROSENKRANZ: "Synergistic renal effects of RS-10085, an ACE inhibitor, and fenoldopam, a DA1 agonist, in the rat" * page 87,88,89 * --- | 1-8 | A 61 K 37/64<br>A 61 K 31/55 //<br>(A 61 K 31/55<br>A 61 K 31:40 ) |
| X | PHARMACEUTICAL NEWS INDEX 88-01637: "New heart failure therapies discussed: Vasodilators ACE-inhibitors" & SCRIP World Pharmaceutical News No 1263, Dec. 4. 1987) p. 26 * whole abstract * --- | 1-3 | |
| X | Pharmaceutical News Index 86-18887: "Blockbusters R-D; Cardiovasculars Mevinolin and eptastatin psychotherapeutics" & SCRIP World Pharmaceutical News No 1104, May 21 1986, p. 24 * Whole abstract * ----- | 1-3 | TECHNICAL FIELDS SEARCHED (Int. Cl.5)<br>A 61 K |

The present search report has been drawn up for all claims

| Place of search | Date of completion of the search | Examiner |
|---|---|---|
| THE HAGUE | 06-02-1990 | PEETERS J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document